# EUROPÄISCHE PATENTSCHRIFT

(11) **EP 3 334 351 B1**
(45) Veröffentlichungstag und Bekanntmachung des Hinweises auf die Patenterteilung: **02.10.2024**
(21) Anmeldenummer: 16757584.4
(22) Anmeldetag: 12.08.2016
(51) Int. Cl.: A61B 17/10, A61B 17/068, A61B 17/072, A61B 17/285, A61B 17/00, A61B 17/32, A61B 17/3211, A61B 18/00, A61B 18/08, A61B 17/29

(54) **VORRICHTUNG ZUR DURCHTRENNUNG VON GEWEBETEILEN**
DEVICE FOR SEPARATING TISSUE PARTS
DISPOSITIF DE COUPE DE PIÈCES DE TISSU

(30) Priorität: 12.08.2015 DE 102015113307
(43) Veröffentlichungstag der Anmeldung: 20.06.2018
(73) Patentinhaber: Charité - Universitätsmedizin Berlin, 10117 Berlin (DE)
(72) Erfinder: FIKATAS, Panagiotis, 10439 Berlin (DE); SAUER, Igor, 10405 Berlin (DE); BAHRA, Marcus, 10113 Berlin (DE)
(74) Vertreter: Gulde & Partner
(86) Internationale Anmeldenummer: PCT/EP2016/069233
(87) Internationale Veröffentlichungsnummer: WO 2017/025626

(56) Entgegenhaltungen:
- WO-A1-2013/130859
- WO-A1-2013/130859
- KR-B1- 101 444 058
- KR-B1- 101 444 058
- US-A1- 2010 030 248
- US-A1- 2010 030 248

## Beschreibung

Die Erfindung betrifft eine Vorrichtung zur Durchtrennung von Gewebeteilen.

Zur Durchtrennung von Gewebeanteile werden in der Chirurgie lineare Stapler eingesetzt. Das Funktionsprinzip besteht in der maximalen Kompression des Gewebes zwischen zwei Metallplatten, Adaptieren der Enden mit tangentialen Klammern und Durchtrennen mit einer mittig verlaufenden Klinge.

Der Einsatz eines linearen Staplers ist aufgrund der Funktionsweise nur bei flachen Gewebestrukturen wie z.B. einem Darm vorteilhaft, sodass sie sich in der Chirurgie des Gastrointerstinaltraktes bewährt haben. Die Durchtrennung von soliden Organen wird zumeist manuell mithilfe von Koagulation der Resektionsflächen durchgeführt. Bei starren und voluminösen Organen (z.B. Leber, Niere, Pankreas, Uterus) zeigte sich, dass lineare Stapler zu Rissen weit über den Resektionsrand hinaus durch Quetschung des Gewebes durch die Metallplatten führten. Dies führt zu intra- bzw. postoperativen Blutungen mit hoher Wahrscheinlichkeit einer Fistelbildung.

Da in der minimalinvasiven Chirurgie nur lineare Stapler zur Verfügung stehen, erfolgte bisher die Resektion von soliden Organen in offener oder Hybrid-Technik. Bei der Durchtrennung solider Organe steht die Vermeidung von Scherkräften durch Quetschungen des Gewebes und der sichere Verschluss der Resektionskante im Vordergrund. Die Resektion großer Gewebestücke ohne die Entstehung von großen Resektionsflächen ist mit der "Fischmaultechnik" möglich. Dabei wird die Resektion nicht linear durchführt, sondern das Gewebe wird keilförmig durchtrennt. Dabei entstehen fischmaulförmige Resektionsränder. Die Adaptation dieser Ränder ermöglicht es die Resektionsfläche komplett nach innen abzudecken. Die Komplexität der Resektion in "Fischmaultechnik" erlaubte bisher nur die Anwendung bei offenen Operationen.

DE 199 51 940 C2 offenbart ein offen und endoskopisch einsetzbares Klammernahtgerät mit einem Betätigungsgriff und einem Klammerkopf, die mit in einem Hohlschaft verlaufenden Bowdenzügen verbunden sind, wobei der Hohlschaft abnehmbar ist.

Weitere Ausführungsformen vergleichbarer Vorrichtungen des Standes der Technik sind offenbart in KR 101 444 058 B1, WO 2013/130859 A1 und US 2010/030248 A1.

Der Erfindung liegt nun die Aufgabe zugrunde, die Durchtrennung auch solider Organe sowohl in offener als auch in minimalinvasiver Chirurgie zu verbessern.

Diese Aufgabe wird mit einer Vorrichtung gemäß Anspruch 1 gelöst. Vorteilhafte Ausgestaltungen finden sich in den abhängigen Ansprüchen.

Die erfindungsgemäße Vorrichtung zur Durchtrennung von Gewebeteilen umfasst zwei parallel verlaufende Klemmplatten, wobei zumindest ein Teil einer Klemmplatte von der anderen Klemmplatte zur Aufnahme eines Gewebeteils weg schwenkbar und zur Fixierung des Gewebeteils zurück schwenkbar ist, mindestens zwei in einem Winkel zueinander angeordnete und verbundene Klingen aufweist, wobei die Klingen in Führungsschienen der Klemmplatten einführbar und verschiebbar sind und wobei eine Schnittkante der Klinge oder Klingen zwischen den Klemmplatten verläuft.

Die erfindungsgemäße Vorrichtung hat den Vorteil, dass eine Durchtrennung auch solider Organe maschinell zu bewerkstelligen ist, ohne dass eine Quetschung des Resektionsrandes mit den oben genannten Risiken stattfindet. Besonders in der minimalinvasiven Chirurgie, bei der bisher lineare Stapler aufgrund fehlender Alternativen verwendet werden, kann der hier beschriebene Stapler Operationsabläufe vereinfachen bzw. das bisherige Operationsspektrum deutlich erweitern. Die Durchtrennung solider Organe mit der erfindungsgemäßen Vorrichtung mit Kantenversiegelung in "Fischmaultechnik" erlaubt es, solide Organe sicher und mit vermindertem Komplikationsrisiko zu durchtrennen. Durch die maschinelle Resektion sinkt signifikant die Operationszeit. Zusätzlich zu der offenen Chirurgie, kann das Gerät auch in der minimalinvasiven Chirurgie Anwendung finden. Besonders bei minimalinvasiven Eingriffen sind die aktuellen Resektionsmöglichkeiten von soliden Organen aufgrund fehlenden Instrumentariums deutlich eingeschränkt. Die hier vorgestellte Vorrichtung könnte minimalinvasive Operationen ermöglichen, die bisher nur in offener Technik durchführbar waren.

Die erfindungsgemäße Vorrichtung kann auch als Stapler oder linearer Stapler bezeichnet werden. Die Klemmplatten haben vorzugsweise eine rechteckförmige und/oder schmale Klemmfläche. Eine oder beide beziehungsweise ein Teil von einer oder beiden Klemmplatten ist schwenkbar ausgestaltet. Die Klingen können fest miteinander verbunden sein, beispielsweise durch einen Schweißvorgang. Der Abstand der Klemmplatten kann einstellbar sein, um an unterschiedliche Gewebeteile angepasst werden zu können.

Die Klingen können direkt in die Führungsschienen einführbar und verschiebbar sein oder fest oder verschiebbar in einem Schneideinsatz angeordnet sein, wobei der Schneideinsatz in die Führungsschienen einführbar und verschiebbar ist. Dies erlaubt eine vielfältige Anpassung der Vorrichtung zum Beispiel an unterschiedliche Einsatzzwecke.

Die zwei Klingen, die jeweils einen Klingenkörper und eine Schneidfläche aufweisen, sind in einem Winkel α zueinander angeordnet. Das heißt der Winkel α wird zwischen den zueinander geneigten Seitenflächen der Klingen der Klingenkörper der beiden Klingen aufgespannt. Alternativ, jedoch nicht im Rahmen der beanspruchten Erfindung, kann auch eine Klinge verwendet werden, die bevorzugt mittig in einem Winkel α geknickt ist. Der Winkel α liegt in einem Bereich zwischen 40° und 150°, vorzugsweise von 90°. Dieser Winkel α erlaubt eine Resektion des Gewebes/Organteils in Fischmaultechnik mit kleinen Resektionsflächen. Das heißt, das zu entfernende Gewebestück/Organteil wird keilförmig aus dem Organ heraus getrennt.

Die Schneidflächen der beiden Klingen können eine Schnittkante bilden. Ein Schnittpunkt entsteht an der Stelle, an der die beiden Schneidflächen der Klingen aufeinander treffen. Dieser Schnittpunkt der beiden Klingen kann fluchtend über den äußeren Enden der beiden Klingen liegen oder auch versetzt vorliegen. Das heißt, die Schnittkante der beiden Klingen kann so angeordnet sein, dass diese einen Winkel β zum Lot durch den Schnittpunkt der Klingen definiert. Der Winkel β kann in einem Bereich von 0 bis 80° liegen. Somit kann der Schnittpunkt der beiden Klingen im Bereich der Klemmplatten oder des Schneideinsatzes zurückgesetzt oder vorgesetzt im Bezug zu den äußeren Flächen der Klingen angeordnet sein. Der Schnittpunkt der beiden Klingen kann auch als Scheitelpunkt der Winkel α und/oder β oder Winkelscheitel bezeichnet werden. Die äußeren Enden der Klingen liegen dann an Endpunkten von Schenkeln des Winkels α. Die äußeren Enden sind in den Führungsschienen der Klemmplatten geführt, was dann zu einer stabileren Durchtrennung mit sauberer Schnittkannte führt, weil die äußeren Enden das Gewebe zuerst durchtrennen. Alternativ kann der Schnittpunkt der beiden Klingen vorgelagert oder vorgesetzt sein, so dass der Schnittpunkt das Gewebe zuerst durchtrennt. Diese Konfiguration kann bei bestimmten Einsatzgebieten von Vorteil sein. Optional kann dann eine stabilere, zum Beispiel dickere, Klinge vorgesehen sein.

Das Gewebe kann bereits durch ein Vorschieben der Klingen in geschlossenem Zustand durchtrennt werden. Weiterhin ermöglicht das Öffnen der Schnittkante, Packen des Gewebes und Schließen (Aufeinandertreffen) der Klingen das Durchtrennen des Gewebes an der Längskante der Klingen.

Der Schneideinsatz kann in dem der Schnittkante abgewandten Bereich Ausnehmungen aufweisen. Diese Ausnehmungen können derart gestaltet sein, dass sie zumindest teilweise in dem Bereich des Schneideinsatzes, der Klingen und/oder eines Grundkörpers des Schneideinsatzes angeordnet sind, der in die Führungsschienen der Klemmplatten eingreift. Dadurch wird der Führungsbereich des Schneideinsatzes verkürzt, was das Einführen und/oder das Entfernen des Schneideinsatzes vereinfacht.

Der Schneideinsatz kann einen Grundkörper aufweisen, der in die Führungsschienen der Klemmplatten einführbar und in diesen verschiebbar ist und der Aufnahmen zur Befestigung oder verschiebbaren Aufnahme der Klinge oder Klingen aufweist. Der Grundkörper kann die Handhabung des Schneideinsatzes verbessern, da dann die Klingen nicht direkt berührt werden müssen.

Die Klingen können einen Stromanschluss für eine thermische Koagulation und/oder für ein Ultraschallgerät für eine thermische Koagulation und/oder Ultraschalldissektion aufweisen. Dies erleichtert den Ablauf der Durchtrennung und verbessert das Resektionsergebnis.

Die Vorrichtung kann eine Klammerpatrone aufweisen mit senkrecht oder zu einem anderen Winkel zu den Klemmplatten angeordneten Klammern. Die Klammern können aus einem metallischen Werkstoff bestehen. Die Klammern können vorgespannt sein, so dass sie nach einer Auslösung die Resektionsränder auch ohne Zuführen weiterer Kräfte verklammern. Alternativ oder zusätzlich kann der Klammereinsatz eine Kraft auf die Klammern ausüben, um diese zu schließen.

Die Klammerpatrone kann direkt in die Führungsschienen einführbar und verschiebbar sein oder fest oder verschiebbar in einem Klammereinsatz angeordnet sein, wobei der Klammereinsatz in die Führungsschienen einführbar und verschiebbar ist. Dies erlaubt eine vielfältige Anpassung der Vorrichtung zum Beispiel an unterschiedliche Einsatzzwecke.

Der Klammereinsatz kann parallel zu den Klemmplatten verlaufende Stifte zum Schließen aufweisen, wobei die Stifte zur Kontaktierung der Klammern verschiebbar angeordnet sind oder die Stifte die Klammern über Schließelemente kontaktieren. Dies erlaubt eine gleichzeitige oder quasigleichzeitige Klammerung in einem Arbeitsschritt, was den Arbeitsvorgang vereinfacht.

Die Klammerpatrone und/oder der Klammereinsatz können mindestens einen Kanal mit Öffnungen zur Bereitstellung einer Flüssigkeit oder eines Granulats aufweisen. Durch die Zugabe oder Injektion von koagulationsfördernden beziehungsweise fibrinbildenden Substanzen kann das Operationsergebnis mittels Senkung der Blutungsneigung und Förderung der Heilung deutlich verbessert werden. Zudem ist es möglich einen Zweikomponenten- (2K) oder Mehrkomponenten-Kleber zur Verklebung oder Verschließung der Resektionsflächen zuzuführen.

Die Vorrichtung kann ein Griffteil aufweisen, an dem die Klemmplatten befestigt sind und dass ein Griff des Griffteils zur Schwenkung des zumindest einen Teils einer Klemmplatte eingerichtet ist. Das Griffteil kann die Form eines Revolvergriffes haben. Das Griffteil erleichtert die Handhabung der Vorrichtung.

Die Klammern können unter Spannung angeordnet sein und nach einer Auslösung selbständig verschließbar sein. Dieses Verschließen ohne äußeren Kraftaufwand kann den Aufbau vereinfachen.

Die Klingen und die Klammerpatrone können axial hintereinander in den Führungsschienen angeordnet sein. Dabei können beide Elemente auch als eine verbundene Einheit vorgesehen sein. Die Klingen und die Klammerpatrone können dann hintereinander durch die Führungsschienen geschoben und vorne aus ihnen herausgeschoben werden.

Der Abstand des schwenkbaren Teils einer Klemmplatte von der anderen Klemmplatte einstellbar sein. So kann zum Beispiel in einem Gelenk, beispielsweise wie bei einer Rohrzange, sowohl der Öffnungswinkel als auch der Abstand der beiden geschlossenen, dann parallel verlaufenden, Klemmplatten eingestellt werden. Dies erlaubt eine Adaption an unterschiedliche Einsatzzwecke.

Im Bereich der zwei parallel verlaufenden Klemmplatten kann ein Gelenk vorgesehen sein. Somit ist die Vorrichtung anpassbar an unterschiedliche Einsatzwinkel. Das Gelenk kann zwischen dem Griff und dem Schwenkgelenk angeordnet sein. Dies ist eine platzsparende Lösung, die dennoch einen großen Arbeitsbereich bietet.

Die Erfindung wird nachfolgend in Ausführungsbeispielen anhand der zugehörigen Zeichnungen erläutert. Es zeigen:
Figur 1 eine schematische Darstellung eines Ausführungsbeispiels einer Vorrichtung zur Durchtrennung von Gewebeteilen,
Figur 2 eine schematische Darstellung eines weiteren Ausführungsbeispiels einer Vorrichtung zur Durchtrennung von Gewebeteilen,
Figur 3 eine Schnittdarstellung der Vorrichtung aus Figur 2,
Figur 4 eine schematische Darstellung eines Schneideinsatzes der Vorrichtung,
Figur 5 eine Schnittdarstellung des Schneideinsatzes aus Figur 2,
Figur 6 eine schematische Darstellung eines Klammereinsatzes der Vorrichtung,
Figur 7 eine erste Schnittdarstellung des Klammereinsatzes aus Figur 6,
Figur 8 eine zweite Schnittdarstellung des Klammereinsatzes aus Figur 6,
Figur 9 eine dritte Schnittdarstellung des Klammereinsatzes aus Figur 6,
Figur 10 eine perspektivische Darstellung der Vorrichtung mit einem fixierten Gewebeteil,
Figur 11 eine perspektivische Darstellung der Vorrichtung mit einem durchtrennten Gewebeteil,
Figur 12 eine perspektivische Darstellung der Vorrichtung mit einem geklammerten Gewebeteil, und
Figuren 13a und 13b eine Seitenansicht einer Klinge z.B. für einen Schneideinsatz der Figur 4.

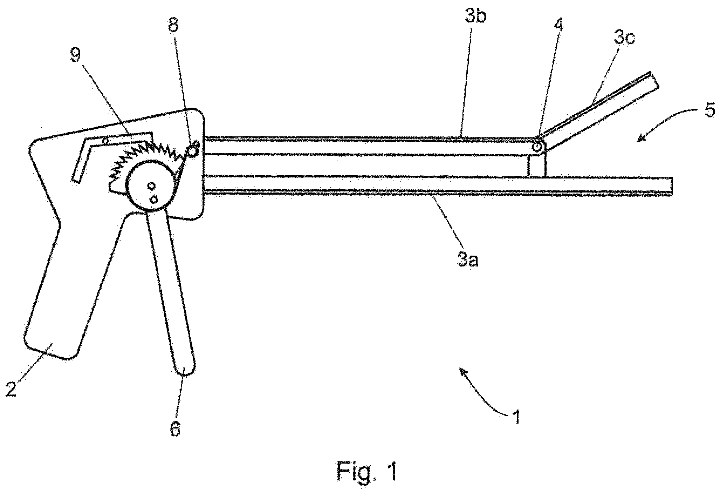

Figur 1 zeigt eine Vorrichtung 1 zur Durchtrennung von Gewebeteilen, insbesondere von Gewebeteilen solider oder starrer Organe, wie z. B. der Leber, der Niere, des Pankreas, des Uterus, usw.. Die Vorrichtung 1 umfasst ein Griffteil 2, an dem zwei parallel verlaufende Klemmplatten 3a und 3b befestigt sind. Zumindest ein Teil oder Bereich 3c einer Klemmplatte, hier der Klemmplatte 3b, ist mittels eines Scharniers 4 schwenkbar gelagert. Eine Drehachse des Scharniers oder Gelenks 4 ist senkrecht zu einer Längserstreckung der Klemmplatten 3a und 3b. Der Teil 3c der Klemmplatte 3b kann von der anderen Klemmplatte 3a zur Aufnahme eines Gewebeteils weggeschwenkt und zur Fixierung des Gewebeteils zurückgeschwenkt werden. Zwischen der Klemmplatte 3a und dem schwenkbaren Teil 3c der anderen Klemmplatte 3b wird somit ein Aufnahmeraum 5 gebildet, der wie bei einer Zange geöffnet und geschlossen werden kann, was durch die Schwenkbewegung des Teils 3c realisiert ist.

Über einen Griff 6 des Griffteils 2 wird das schwenkbare Teil 3c bewegt. Der Griff 6 und der schwenkbare Teil 3c sind beispielsweise über einen Bowdenzug 8 oder mindestens einer Metallstrebe miteinander verbunden. Ein Rast- oder Arretiermechanismus 9 kann den schwenkbaren Teil 3c in jeder Stellung arretieren. Der Arretiermechanismus 9 ist wieder lösbar ausgestaltet.

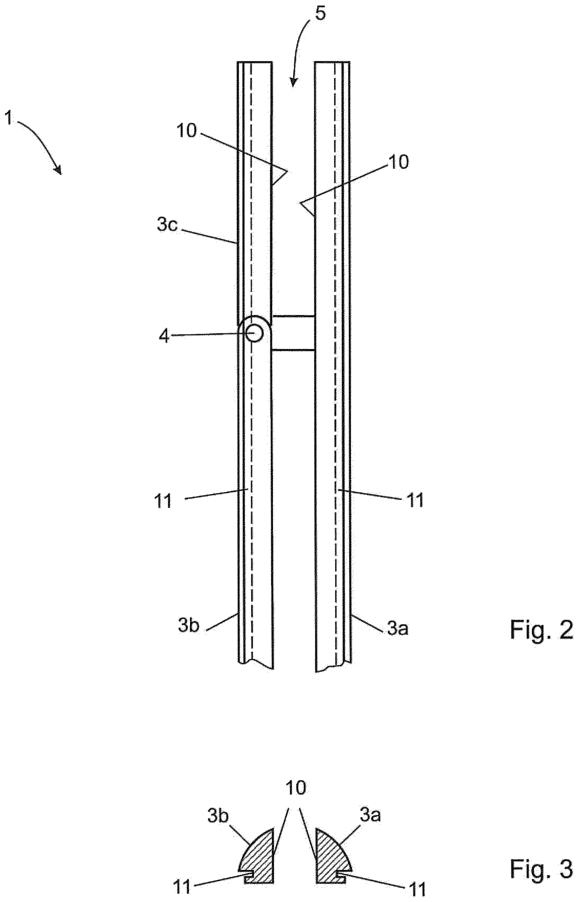

Anhand der Figuren 2 und 3 werden nun die Klemmplatten 3a und 3b, die auch als Staplerkopf oder Teil eines Staplerkopfes bezeichnet werden können, genauer beschrieben. In Figur 2 ist der Staplerkopf mit den beiden Klemmplatten 3a und 3b in geschlossener Stellung gezeigt. Somit verlaufen die beiden Klemmplatten 3a und 3b auch in dem Klemmbereich 5 parallel. Jede Klemmplatte 3a und 3b umfasst eine Klemmfläche 10, wobei die beiden Klemmflächen 10 der beiden Klemmplatten 3a und 3b einander zugewandt sind. Mittels dieser Klemmflächen 10 kommt es im geschlossenen Zustand zu einer definierten, jedoch nicht völligen Kompression von Gewebe, das sich im Klemmbereich 5 zwischen den beiden Klemmplatten 3a und 3b befindet. Die Klemmung dient nicht zur Durchtrennung oder anfänglichen Durchtrennung des Gewebes, sondern nur zur Fixierung des Gewebes in dem Staplerkopf. Die Klemmflächen 10 und/oder die Klemmplatten 3a und 3b haben eine längliche bzw. rechteckförmige Form. Sie sind schmal ausgestaltet, das heißt, dass die Breite klein ist im Vergleich zu der Länge.

Jede Klemmplatte 3a und 3b umfasst eine Führungsschiene 11, die parallel zueinander verlaufen. Der Begriff parallel umschließt in dieser Beschreibung auch den geöffneten Zustand der Vorrichtung 1, das heißt auch den Fall, dass der Teil 3c der Klemmplatte 3b aufgeschwenkt und damit nicht tatsächlich parallel zu der anderen Klemmplatte 3a verläuft. Da jedoch der restliche Teil der Klemmplatte 3b und der auch der Teil 3c im geschlossenen Zustand parallel verläuft, wird hier im Rahmen dieser Beschreibung der Begriff parallel verwendet.

Die Führungsschienen 11 sind an den Klemmflächen 10 abgewandten Flächen der Klemmplatten 3a und 3b angeordnet. Alternativ können sie an Flächen ausgebildet sein, welche an die Klemmflächen 10 angrenzen.

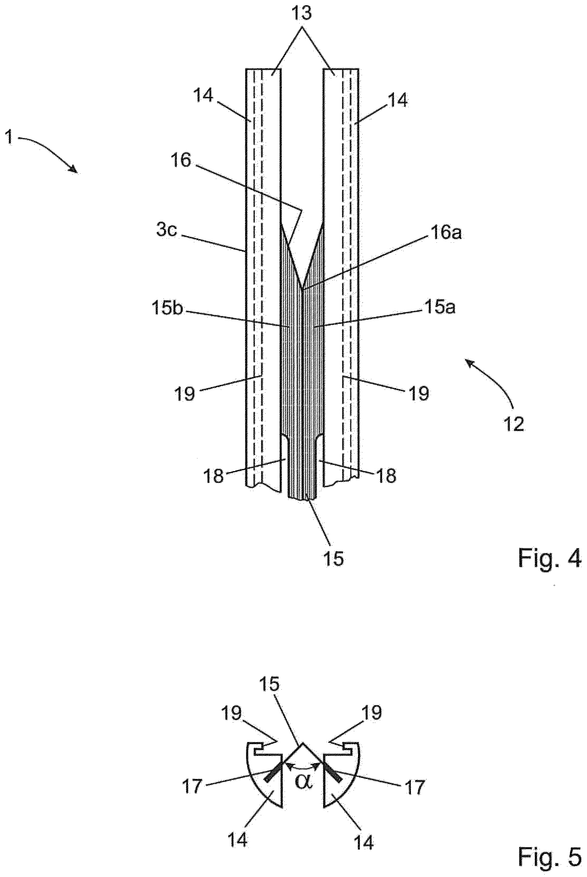

Anhand der Figuren 4 und 5 wird nun ein Schneideinsatz der Vorrichtung 1 näher beschrieben, der in die Führungsschienen 11 der Klemmplatten 3a und 3b eingeführt werden kann und in diesen verschoben werden kann. Der Schneideinsatz 12 umfasst ein Basisteil oder Grundkörper 13 mit zwei parallel verlaufenden Platten 14. Zwischen den Platten 14 ist eine Klinge 15 vorgesehen, welche aus zwei in einem Winkel zueinander angeordneten und miteinander verbundenen Klingen 15a und 15b besteht. Die Klinge 15 kann einteilig ausgebildet sein oder aus den beiden Klingen 15a und 15b zusammengefügt sein. Zur Griffseite 2 sind die beiden Platten 14 fest miteinander verbunden. Die Klinge 15 bzw. die Klingen 15a und 15b haben eine Schnittkante 16 zur Durchtrennung von Gewebe, das in dem Klemmbereich 5 der Vorrichtung 1 angeordnet ist.

Wie in Figur 5 zu sehen ist, ist die Klinge 15 in Aufnahmen 17 der Platten 14 befestigt oder axial beweglich. Die Befestigung kann durch eine Klemmung und/oder Verklebung in den Aufnahmen 17 erfolgen. Die beiden Klingen 15a und 15b sind in einem Winkel α zueinander angeordnet. Der Winkel α hat hier einen Wert zwischen 60° und 120° und hat vorzugsweise einen Wert von 90°. Der Winkel α und die Klinge 15 sind derart ausgebildet, dass die Klinge 15 V-förmig zwischen den beiden Platten 14 angeordnet ist. In der in den Figuren 4 und 5 dargestellten Orientierung zeigt die Klinge nach oben. Das heißt, dass der Schnittpunkt der beiden Klingen 15a und 15b je nach Winkel α und Längen der Klingen 15a und 15b unterhalb oder oberhalb der Aufnahmen 17 bzw. der Platten 14 liegt.

Bei der Schnittkante 16 ist ein Schnittpunkt 16a der beiden Klingen 15a und 15b zurückgesetzt im Vergleich zu den äußeren Enden der beiden Klingen 15a und 15b, die in den Aufnahmen 17 der Platten 14 angeordnet sind. Der Begriff zurückgesetzt bezieht sich auf eine axiale oder Längsrichtung des Schneideinsatzes 12 bzw. der Vorrichtung 1. Die Längsrichtung verläuft in Richtung der parallel verlaufenden Klemmplatten 3a und 3b bzw. der Platten 14. An einer hinteren Seite oder einem der Schnittkante 16 abgewandten Bereich hat der Schneideinsatz 12 bzw. die Klinge 15 und/oder die Platte 14 Ausnehmungen 18. Anhand dieser Ausnehmungen 18 kann die Klinge 15 leichter aus dem Schneideinsatz 12 bzw. der Schneideinsatz 12 leichter aus den Klemmplatten 3a und 3b entfernt werden.

Zum Einführen des Schneideinsatzes in die Klemmplatten 3a und 3b enthalten die Platten 14 des Schneideinsatzes 12 Führungsschienen 19, die komplementär zu den Führungsschienen 11 der Klemmplatten 3a und 3b ausgeführt sind. Dies bedeutet, dass die Führungsschienen 11 und 19 ineinander greifen können, so dass der Schneideinsatz 12 in den Klemmplatten 3a und 3b verschiebbar geführt ist. Der Schneideinsatz 12 und insbesondere die Klinge 15 bzw. die Aufnahmen 17 sind derart ausgebildet, dass die Klinge 15 bei einem axialen Verschieben des Schneideinsatzes 12 in den Klemmplatten 3a und 3b in den Klemmbereich 5 eingreift.

Optional ist ein Stromanschluss der Klinge 15 bzw. der Klingen 15a und 15b vorgesehen, der für eine thermische Koagulation während des Durchschneidens des Gewebes sorgt.

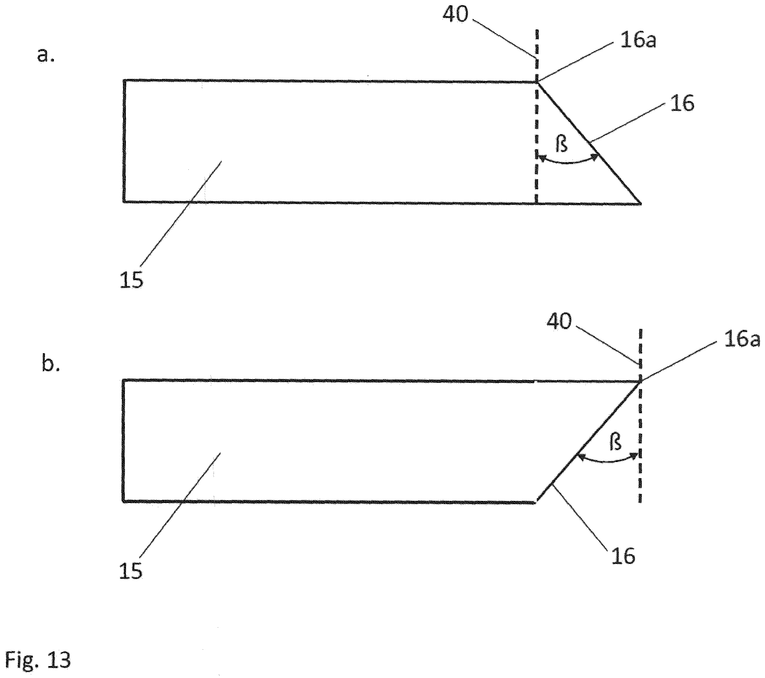

Die Figuren 13a und 13b zeigen eine zweiteilige Klinge 15 für einen Schneideinsatz der Vorrichtung 1 jeweils in einer Seitenansicht. Die Schneidflächen der beiden Klingen bilden die Schnittkante 16, wobei der Schnittpunkt 16a sich an der Stelle befindet, an der die Schneidflächen der beiden Klingen aufeinander treffen. Die Schnittkante 16 ist in einem Winkel β zum Lot 40 durch den Schnittpunkt 16a angeordnet. In Figur 13a ist dargestellt, wie durch den Winkel β der Schnittpunkt 16a der zweiteiligen Klinge 15 relativ zum äußeren Ende der Klinge 15 zurückgesetzt vorliegt. Der Begriff "zurückgesetzt" bezieht sich hier auf eine axiale oder Längsrichtung des Schneideinsatzes 12 der Figur 4. Figur 13b zeigt eine zweiteilige Klinge 15 mit einem Winkel β, wodurch der Schnittpunkt 16a das äußere Ende der Klinge 15 bildet.

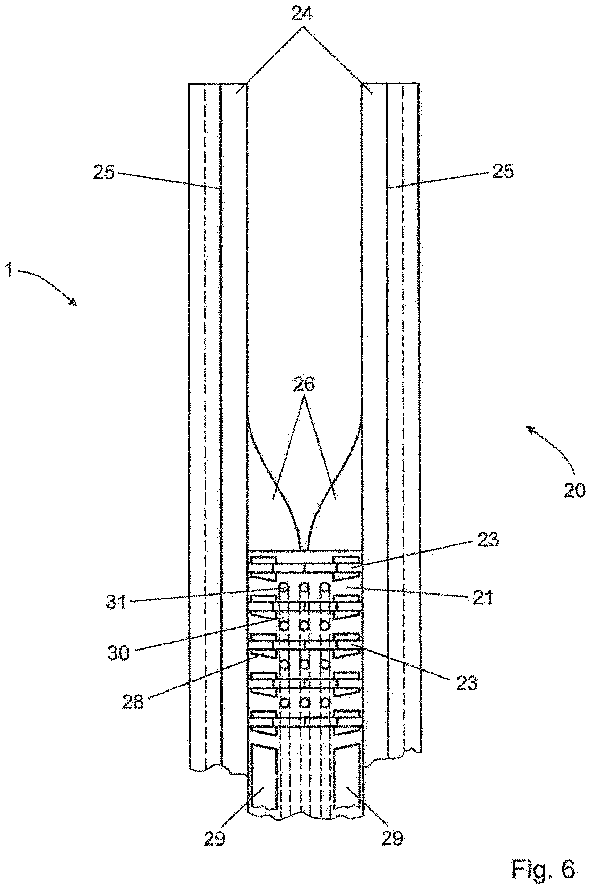

Anhand der Figuren 6 bis 9 wird ein optionaler Klammereinsatz 20 der Vorrichtung 1 beschrieben, der wie der Schneideinsatz 12 in die Klemmplatten 3a und 3b einführbar und in diesen verschiebbar ist. Der Klammereinsatz 20 enthält eine Klammerpatrone 21 mit Klammern 23 zur Verklammerung von Resektionsflächen oder Resektionsrändern von Gewebeteilen, die sich im Klemmbereich 5 der Vorrichtung 1 befinden. Die Klammerpatrone 21 ist zwischen Platten 24 gehalten bzw. verbindet diese. Die Platten 24 entsprechen den Platten 14 des Schneideinsatzes 12. Entsprechend weisen die Platten 24 Führungsschienen 25 auf, welche den Führungsschienen 19 des Schneideinsatzes 12 entsprechen und komplementär zu den Führungsschienen 11 der Klemmplatten 3a und 3b ausgeführt sind. Somit kann der Klammereinsatz 20 in die Klemmplatten 3a und 3b eingeführt werden und in diesen verschoben werden.

Zwei oder auch mehrere der Klammern 23 können miteinander verbunden wie zum Beispiel bei einer Haarklammer. Das heißt, sie können ineinander greifen oder mittels eines oder mehrerer Gelenke verbunden sein.

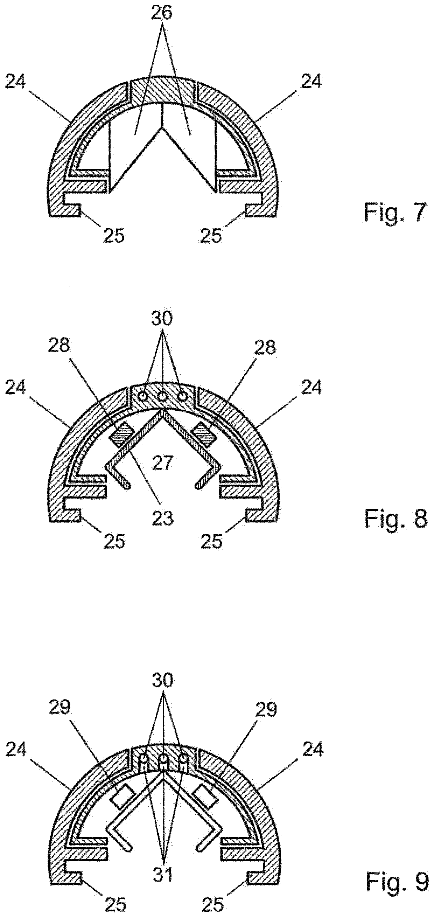

In einem vorderen Bereich, das heißt dem Klemmbereich 5 zugewandt, enthält der Klammereinsatz 20 zwei Adaptationsflächen 26, die zur Zusammenführung der Gewebekanten nach der keilförmigen Resektion dienen. Die Adaptationsflächen 26 bzw. der Innenraum oder der Raum zwischen den beiden Adaptationsflächen 26 sind trichter- bzw. keilförmig ausgeführt, um die Gewebekanten zusammenzuführen. Dieser Querschnitt ist in Figur 7 dargestellt.

Hinter den Adaptationsflächen 26 ist die Klammerpatrone 21 angeordnet. Direkt auf die Adaptationsflächen 26 folgt eine erste Klammer 23 der Klammerpatrone 21. Dieser Querschnitt ist in Figur 8 dargestellt. Die Klammer 23 ist über einen Innenraum 27 aufgespannt, der dem Innenraum zwischen den Adaptationsflächen 26 nachgelagert ist, so dass das durch die Adaptationsflächen 26 zusammengeführte Gewebe in dem Innenraum 27 und damit zwischen der Klammer 23 liegt. An den beiden Schenkeln einer Klammer 23 ist jeweils ein quaderförmiges Schließelement 28 angeordnet. Das Schließelement 28 wird jeweils durch ein axial verlaufenden Stift 29 kontaktiert, wodurch die beiden Schließelemente 28 durch Druck auf die Schenkel der Klammer 23 die Klammer 23 schließen. Alternativ können die Stifte 29 direkt auf die Klammern 23 wirken.

Die Klingen15a und 15b und die Klammerpatrone 21 können axial hintereinander verlaufend angeordnet sein. Des Weiteren kann die Klinge beziehungsweise die Klingen 15a und 15b fest mit den Führungsschienen 11 verbunden sein und der Schneideinsatz 12 kann sich während der Durchtrennung axial komplett bewegen. Es kann weiter vorgesehen sein, dass die Klammerpatrone 21 fest mit den Platten 24 beziehungsweise den Führungsschienen 25 verbunden ist und während des Abclippens oder Verklammerns komplett entlang an den Führungsschienen 11 vorgeschoben wird. Auch können die Schienen 14 und 24 als fester Bestandteil der Klemmplatten 3a und 3b ausgebildet sein, in denen die Klingen 15a und 15b und die Klammerpatrone 21 als Module verlaufen.

Der Klammereinsatz 20 umfasst ferner mehrere Kanäle 30 mit Öffnungen 31 zur Bereitstellung einer Flüssigkeit, wie einer koagulationsfördernden bzw. fibrinbildenden Flüssigkeit. Die Kanäle 30, in diesem Beispiel sind drei Kanäle 30 ausgebildet, es können jedoch ein bis etwa acht Kanäle ausgebildet sein, verlaufen in axialer Richtung, das heißt, parallel zu den Platten 24. Die Öffnungen 31 sind senkrecht zu der axialen Richtung ausgebildet und erlauben eine Zufuhr der Flüssigkeit zu den Resektionsflächen. Die Öffnungen 31 sind in der Klammerpatrone 21 jeweils zwischen zwei Klammern 23 angeordnet, wodurch eine gute Verteilung der Flüssigkeit gewährleistet wird. Dieser Querschnitt ist in Figur 9 dargestellt.

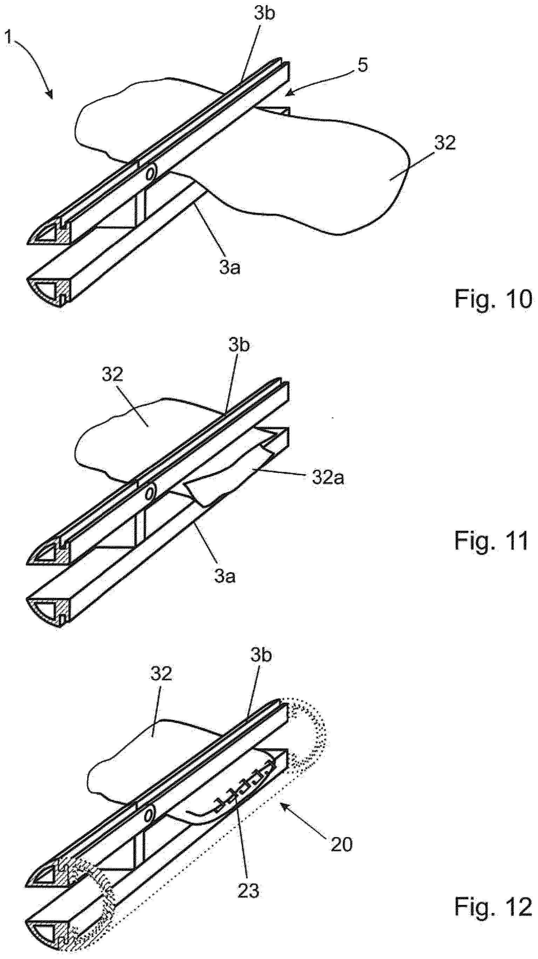

Im Folgenden wird anhand der Figuren 10 bis 12 die Handhabung der Vorrichtung 1 zur Durchtrennung von Gewebeteilen beschrieben.

In Figur 10 ist das Aufklemmen eines Gewebeteils 32 ohne Quetschung zwischen den beiden Klemmplatten 3a und 3b der Vorrichtung 1 gezeigt. Nun ist das Gewebeteil 32 fixiert, jedoch nicht völlig komprimiert.

Nun wird der Schneideinsatz 12 mit seinen Platten 14 in die Führungsschienen 11 der Klemmplatten 3a und 3b eingeführt und in Richtung des Gewebeteils 32 bzw. des Klemmbereichs 5 axial nach vorne geführt. Der Schneideinsatz 12 kann entweder manuell verschoben oder mittels des Griffes 6 oder eines weiteren Betätigungselements des Griffteils 2 in Richtung des Klemmbereichs 5 bewegt werden. Mit der Bewegung des Schneideinsatzes 12 wird die Schnittkante 16 in Richtung des Klemmbereichs 5 bewegt. Die dreieckig geformte und zwischen den beiden Platten 14 aufgespannte Klinge 15 durchtrennt nun durch axiales Vorschieben das Gewebeteil 32 keilförmig.

Die keilförmigen Gewebekanten 32a sind in Figur 11 dargestellt. Nach der Durchtrennung des Gewebes bzw. des Gewebeteils 32 ist der verbleibende Organanteil weiterhin zwischen den Klemmplatten 3a und 3b fixiert. Der Schneideinsatz 12 wird axial herausgeführt und die Resektionsflächen kann inspiziert bzw. manuell zusätzlich versorgt werden.

Nachdem der Schneideinsatz 12 aus den Klemmplatten 3a und 3b entfernt ist, wird der Klammereinsatz 20 in die Klemmplatten 3a und 3b eingeführt. Der Klammereinsatz 20 wird so weit axial vorgeschoben, bis die Klammerpatrone 21 und damit die Klammern 23 in dem Klemmbereich 5 und damit an bzw. über dem Gewebeteil 32 angeordnet sind.

Die Adaptationsflächen 12 des Klammereinsatzes 20 führen bei der Vorwärtsbewegung die Gewebekanten 32a nach der keilförmigen Resektion zusammen, wobei die Klammern 23 über den zusammengeführten Gewebekanten 32 aufgespannt sind. Dieser Zustand ist in Figur 12 dargestellt. Zum Klammern werden nun die Stifte 29 axial nach vorne geschoben, so dass über die Schließelemente 28 die Klammern 23 schließen. Gleichzeitig erfolgt über die Kanäle 30 die Injektion von koagulationsfördernden bzw. fibrinbildenden Flüssigkeiten. Das Ergebnis ist ein komplettes Einstülpen der Resektionsfläche mit Versiegelung potentieller Erosionsstellen.

Anschließend wird der Klammereinsatz 20 in axialer Richtung aus den Klemmplatten 3a und 3b entfernt. Nun wird der schwenkbare Teil 3c der Klemmplatte 3b geöffnet und somit das verbleibende Organ oder Gewebeteil 32 freigegeben.

## Patentansprüche

1. Vorrichtung zur Durchtrennung von Gewebeteilen (32), wobei die Vorrichtung (1) zwei parallel verlaufende Klemmplatten (3a, 3b) aufweist, wobei beide Klemmplatten (3a, 3b) jeweils eine Führungsschiene (11) aufweisen und wobei ein Teil (3c) einer Klemmplatte (3b) von der anderen Klemmplatte (3a) zur Aufnahme eines Gewebeteils (32) weg schwenkbar und zur Fixierung des Gewebeteils (32) zurück schwenkbar ist, **dadurch gekennzeichnet, dass** die Vorrichtung (1) mindestens zwei in einem Winkel α zwischen 40°-150° zueinander angeordnete und verbundene Klingen (15a, 15b) aufweist, wobei der Winkel α zwischen den zueinander geneigten Seitenflächen der beiden Klingen aufgespannt wird, wobei jede der beiden Klingen (15a, 15b) in jeweils eine der beiden Führungsschienen (11) der Klemmplatten (3a, 3b) einführbar und verschiebbar ist und wobei eine Schnittkante (16) der Klingen (15a, 15b) zwischen den Klemmplatten (3a, 3b) verläuft.

2. Vorrichtung nach Anspruch 1, **dadurch gekennzeichnet, dass** die Klingen (15a, 15b) direkt in die Führungsschienen (11) einführbar und verschiebbar sind oder fest oder verschiebbar in einem Schneideinsatz (12) angeordnet sind, wobei der Schneideinsatz (12) in die Führungsschienen (11) einführbar und verschiebbar ist.

3. Vorrichtung nach Anspruch 1 oder 2, **dadurch gekennzeichnet, dass** die zwei Klingen (15a, 15b) in einem Winkel α von 90°, zueinander angeordnet sind.

4. Vorrichtung nach einem der vorhergehenden Ansprüche, **dadurch gekennzeichnet, dass** bei der Schnittkante (16) ein Schnittpunkt (16a) der beiden Klingen (15a, 15b) zurückgesetzt oder vorgesetzt ist zu äußeren Enden der beiden Klingen (15a, 15b).

5. Vorrichtung nach Anspruch 2, oder einem der Ansprüche 3 oder 4, wenn rückbezogen auf Anspruch 2, **dadurch gekennzeichnet, dass** der Schneideinsatz (12) in dem der Schnittkante (16) abgewandten Bereich Ausnehmungen (18) aufweist.

6. Vorrichtung nach einem der Ansprüche 2 bis 5, **dadurch gekennzeichnet, dass** der Schneideinsatz (12) einen Grundkörper (13) aufweist, der in die Führungsschienen (11) der Klemmplatten (3a, 3b) einführbar und in diesen verschiebbar ist und der Aufnahmen (17) zur Befestigung oder verschiebbaren Aufnahme der Klingen (15a, 15b) aufweist.

7. Vorrichtung nach einem der vorhergehenden Ansprüche, **dadurch gekennzeichnet, dass** die Klingen (15a, 15b) einen Stromanschluss und/oder einen Anschluss für ein Ultraschallgerät für eine thermische Koagulation und/oder Ultraschalldissektion aufweisen.

8. Vorrichtung nach einem der vorhergehenden Ansprüche, **dadurch gekennzeichnet, dass** die Vorrichtung (1) eine Klammerpatrone (21) aufweist mit senkrecht oder zu einem anderen Winkel zu den Klemmplatten (3a, 3b) angeordneten Klammern (23).

9. Vorrichtung nach Anspruch 8, **dadurch gekennzeichnet, dass** die Klammerpatrone (21) direkt in die Führungsschienen (11) einführbar und verschiebbar ist oder fest oder verschiebbar in einem Klammereinsatz (20) angeordnet ist, wobei der Klammereinsatz (20) in die Führungsschienen (11) einführbar und verschiebbar ist.

10. Vorrichtung nach Anspruch 9, **dadurch gekennzeichnet, dass** der Klammereinsatz (20) parallel zu den Klemmplatten (3a, 3b) verlaufende Stifte (29) zum Schließen aufweist, wobei die Stifte (29) zur Kontaktierung der Klammern (23) verschiebbar angeordnet sind oder die Stifte (29) die Klammern (23) über Schließelemente (28) kontaktieren.

11. Vorrichtung nach einem der Ansprüche 8 bis 10, **dadurch gekennzeichnet, dass** die Klammerpatrone (21) und/oder der Klammereinsatz (20) mindestens einen Kanal (30) mit Öffnungen (31) zur Bereitstellung einer Flüssigkeit oder eines Granulats aufweist.

12. Vorrichtung nach einem der Ansprüche 1 bis 11, **dadurch gekennzeichnet, dass** die Vorrichtung (1) ein Griffteil (2) aufweist, an dem die Klemmplatten (3a, 3b) befestigt sind und dass ein Griff (6) des Griffteils (2) zur Schwenkung des einen Teils (3c) einer Klemmplatte (3b) eingerichtet ist.

13. Vorrichtung nach einem der Ansprüche 8 bis 12, **dadurch gekennzeichnet, dass** die Klammern (23) unter Spannung angeordnet sind und nach einer Auslösung selbständig verschließbar sind.

14. Vorrichtung nach einem der Ansprüche 8 bis 13, **dadurch gekennzeichnet, dass** die Klingen (15a, 15b) und die Klammerpatrone (21) axial hintereinander in den Führungsschienen (11) angeordnet sind.

15. Vorrichtung nach einem der Ansprüche 1 bis 14, **dadurch gekennzeichnet, dass** der Abstand des schwenkbaren Teils (3c) einer Klemmplatte (3b) von der anderen Klemmplatte (3a) einstellbar ist.

16. Vorrichtung nach einem der Ansprüche 1 bis 15, **dadurch gekennzeichnet, dass** im Bereich der zwei parallel verlaufenden Klemmplatten (3a, 3b) ein Gelenk vorgesehen ist, so dass die Vorrichtung an unterschiedliche Einsatzwinkel anpassbar ist.

## Claims

1. A device for separating tissue parts (32), wherein the device (1) has two clamping plates (3a, 3b) extending in parallel, wherein both clamping plates (3a, 3b) each have a guide rail (11) and wherein a part (3c) of one clamping plate (3b) can be pivoted away from the other clamping plate (3a) for receiving a tissue part (32) and can be pivoted back for fixing the tissue part (32), **characterized in that** the device (1) has at least two blades (15a, 15b) which are arranged and connected to one another at an angle α of between 40°-150°, wherein the angle α is formed between the mutually inclined side surfaces of the two blades, wherein each of the two blades (15a, 15b) is insertable and displaceable in one of the two guide rails (11) of the clamping plates (3a, 3b) and wherein a cutting edge (16) of the blades (15a, 15b) extends between the clamping plates (3a, 3b).

2. The device according to Claim 1, **characterized in that** the blades (15a, 15b) are directly insertable and displaceable in the guide rails (11) or are arranged securely or displaceably in a cutting insert (12), wherein the cutting insert (12) is insertable and displaceable in the guide rails (11).

3. The device according to Claim 1 or 2, **characterized in that** the two blades (15a, 15b) are arranged at an angle α of 90° to one another.

4. The device according to any one of the preceding claims, **characterized in that** at the cutting edge (16) an intersection point (16a) of the two blades (15a, 15b) is set back or forward to the outer ends of the two blades (15a, 15b).

5. The device according to Claim 2, or any one of Claims 3 or 4, when dependent on Claim 2, **characterized in that** the cutting insert (12) has recesses (18) in the region facing away from the cutting edge (16).

6. The device according to any one of Claims 2 to 5, **characterized in that** the cutting insert (12) has a base body (13), which is insertable in the guide rails (11) of the clamping plates (3a, 3b) and is displaceable therein, and which has mounts (17) for securing or displaceably receiving the blades (15a, 15b).

7. The device according to any one of the preceding claims, **characterized in that** the blades (15a, 15b) have a power connection and/or a connection for an ultrasound device for thermal coagulation and/or ultrasonic dissection.

8. The device according to any one of the preceding claims, **characterized in that** the device (1) has a clamp cartridge (21) with clamps (23) arranged perpendicular or at a different angle to the clamping plates (3a, 3b).

9. The device according to Claim 8, **characterized in that** the clamp cartridge (21) is directly insertable and displaceable in the guide rails (11) or is arranged securely or displaceably in a clamp insert (20), wherein the clamp insert (20) is insertable and displaceable in the guide rails (11).

10. The device according to Claim 9, **characterized in that** the clamp insert (20) has pins (29) extending parallel to the clamping plates (3a, 3b) for closing, wherein the pins (29) are arranged displaceably for contacting the clamps (23) or the pins (29) contact the clamps (23) via closing elements (28).

11. The device according to any one of Claims 8 to 10, **characterized in that** the clamp cartridge (21) and/or the clamp insert (20) has at least one channel (30) with openings (31) for providing a liquid or a granulate.

12. The device according to any one of Claims 1 to 11, **characterized in that** the device (1) has a handle part (2) to which the clamping plates (3a, 3b) are secured and **in that** a handle (6) of the handle part (2) is configured for pivoting one part (3c) of a clamping plate (3b).

13. The device according to any one of Claims 8 to 12, **characterized in that** the clamps (23) are arranged under tension and are automatically closable after release.

14. The device according to any one of Claims 8 to 13, **characterized in that** the blades (15a, 15b) and the clamp cartridge (21) are arranged axially behind one another in the guide rails (11).

15. The device according to any one of Claims 1 to 14, **characterized in that** the distance of the pivotable part (3c) of one clamping plate (3b) from the other clamping plate (3a) is adjustable.

16. The device according to any one of Claims 1 to 15, **characterized in that** a joint is provided in the region of the two clamping plates (3a, 3b) extending in parallel so that the device is adaptable to different insertion angles.

## Revendications

1. Dispositif de coupe de pièces de tissu (32), le dispositif (1) présentant deux plaques de serrage (3a, 3b) s'étendant de façon parallèle, les deux plaques de serrage (3a, 3b) présentant respectivement un rail de guidage (11) et une partie (3c) d'une plaque de serrage (3b) pouvant s'écarter de l'autre plaque de serrage (3a) en pivotant pour la réception d'une pièce de tissu (32) et pouvant retourner en pivotant pour la fixation de la pièce de tissu (32), **caractérisé en ce que** le dispositif (1) présente au moins deux lames (15a, 15b) disposées avec un angle α de 40°-150° l'une par rapport à l'autre et reliées, l'angle α étant formé entre les surfaces latérales des deux lames inclinées l'une vers l'autre, chacune des deux lames (15a, 15b) pouvant être insérée et déplacée dans l'un des deux rails de guidage (11) des plaques de serrage (3a, 3b), respectivement, et une arête de coupe (16) des lames (15a, 15b) s'étendant entre les plaques de serrage (3a, 3b).

2. Dispositif selon la revendication 1, **caractérisé en ce que** les lames (15a, 15b) peuvent directement être insérées et déplacées dans les rails de guidage (11) ou sont disposées de façon fixe ou déplaçable dans un élément de coupe (12), l'élément de coupe (12) pouvant être inséré et déplacé dans les rails de guidage (11).

3. Dispositif selon la revendication 1 ou 2, **caractérisé en ce que** les deux lames (15a, 15b) sont disposées avec un angle α de 90° l'une par rapport à l'autre.

4. Dispositif selon l'une quelconque des revendications précédentes, **caractérisé en ce que**, sur l'arête de coupe (16), une intersection (16a) des deux lames (15a, 15b) est située en arrière ou en avant par rapport à des extrémités extérieures des deux lames (15a, 15b).

5. Dispositif selon la revendication 2 ou selon l'une quelconque des revendications 3 ou 4 lorsqu'elles se rapportent à la revendication 2, **caractérisé en ce que** l'élément de coupe (12) présente des évidements (18) dans la zone opposée à l'arête de coupe (16).

6. Dispositif selon l'une quelconque des revendications 2 à 5, **caractérisé en ce que** l'élément de coupe (12) présente un corps de base (13), lequel peut être inséré dans les rails de guidage (11) des plaques de serrage (3a, 3b) et est déplaçable dans celles-ci, et présente des logements (17) pour la fixation ou la réception de manière déplaçable des lames (15a, 15b).

7. Dispositif selon l'une quelconque des revendications précédentes, **caractérisé en ce que** les lames (15a, 15b) présentent une connexion électrique et/ou une connexion pour un appareil à ultrasons pour une coagulation thermique et/ou une dissection par ultrasons.

8. Dispositif selon l'une quelconque des revendications précédentes, **caractérisé en ce que** le dispositif (1) présente une cartouche d'agrafes (21) avec des agrafes (23) disposées de façon verticale ou avec un autre angle par rapport aux plaques de serrage (3a, 3b).

9. Dispositif selon la revendication 8, **caractérisé en ce que** la cartouche d'agrafes (21) peut directement être insérée et déplacée dans les rails de guidage (11) ou est disposée de façon fixe ou déplaçable dans un élément d'agrafes (20), l'élément d'agrafes (20) pouvant être inséré et étant déplaçable dans les rails de guidage (11).

10. Dispositif selon la revendication 9, **caractérisé en ce que** l'élément d'agrafes (20) présente des broches (29) s'étendant parallèlement aux plaques de serrage (3a, 3b) pour la fermeture, les broches (29) étant disposées de façon déplaçable pour l'établissement d'un contact avec les agrafes (23) ou les broches (29) établissant un contact avec les agrafes (23) par des éléments de fermeture (28).

11. Dispositif selon l'une quelconque des revendications 8 à 10, **caractérisé en ce que** la cartouche d'agrafes (21) et/ou l'élément d'agrafes (20) présentent au moins un canal (30) avec des ouvertures (31) pour la mise à disposition d'un liquide ou d'un granulat.

12. Dispositif selon l'une quelconque des revendications 1 à 11, **caractérisé en ce que** le dispositif (1) présente une partie de préhension (2), sur laquelle les plaques de serrage (3a, 3b) sont fixées, et **en ce qu'**une poignée (6) de la partie de préhension (2) est conçue pour le pivotement d'une partie (3c) d'une plaque de serrage (3b).

13. Dispositif selon l'une quelconque des revendications 8 à 12, **caractérisé en ce que** les agrafes (23) sont disposées sous tension et peuvent être fermées de façon autonome après un déclenchement.

14. Dispositif selon l'une quelconque des revendications 8 à 13, **caractérisé en ce que** les lames (15a, 15b) et la cartouche d'agrafes (21) sont disposées les unes derrière les autres de façon axiale dans les rails de guidage (11).

15. Dispositif selon l'une quelconque des revendications 1 à 14, **caractérisé en ce que** la distance de la partie pivotante (3c) d'une plaque de serrage (3b) à l'autre plaque de serrage (3a) peut être réglée.

16. Dispositif selon l'une quelconque des revendications 1 à 15, **caractérisé en ce qu'**une articulation est prévue dans la zone des deux plaques de serrage (3a, 3b) s'étendant de façon parallèle, de telle sorte que le dispositif peut être ajusté à différents angles d'utilisation.
